Europäisches Patentamt

European Patent Office

Office européen des brevets

(19)

(11) Publication number: **0 109 559**
**A1**

(12) # EUROPEAN PATENT APPLICATION

(21) Application number: **83110471.6**

(51) Int. Cl.³: **C 12 N 15/00**

(22) Date of filing: **20.10.83**

(30) Priority: **20.10.82 JP 184291/82**

(43) Date of publication of application:
**30.05.84 Bulletin 84/22**

(84) Designated Contracting States:
**AT BE CH DE FR GB IT LI LU NL SE**

(71) Applicant: **SUNTORY KABUSHIKI KAISHA, also known as SUNTORY LTD.**
**1-40, Dojimahama 2-chome**
**Kita-ku Osaka(JP)**

(72) Inventor: **Oshima, Takehiro**
**17-10-383, Besshohonmachi**
**Takatsuki-shi Osaka(JP)**

(72) Inventor: **Tanaka, Shoji**
**8-46, Minamikaneden-cho 1-chome**
**Suita-shi Osaka(JP)**

(72) Inventor: **Nakazato, Hiroshi**
**13-E-505, Misawa-cho**
**Ibaraki-shi Osaka(JP)**

(74) Representative: **Hansen, Bernd, Dr.rer.nat. et al,**
**Hoffmann, Eitle & Partner Patentanwälte Arabellastrasse 4**
**D-8000 München 81(DE)**

(54) **High-efficiency yeast vector plasmid and method of its use.**

(57) A yeast vector having a gene for the target peptide combined with a promoter for an operon which controls glyceraldehyde-3-phophate dehydrogenase which is one of the enzymes taking part in the glycolysis of a yeast strain, Saccharomyces, a process for preparing the vector and a process for producing the target peptide from yeast in high yield by using such vector.

EP 0 109 559 A1

# HIGH-EFFICIENCY YEAST VECTOR PLASMID AND METHOD OF ITS USE

The present invention relates to a yeast vector having a gene for the target peptide combined with a promoter for a gene (operon) which controls glyceraldehyde-3-phosphate dehydrogenase (hereunder referred to as GAP-DH) which is one of the enzymes taking part in the glycolysis of a yeast strain, Saccharomyces. More particularly, the invention relates to a process for producing the target peptide from yeast in high yield by using such promoter.

The recent development of the technology of genetic engineering is so remarkable that today, pharmaceutically useful peptides such as somatostatin, insulin, growth hormones, interferons (alpha, beta and gamma), influenza viruses, hepatitis B viral protein, thymosin alpha 1, beta-endorphin, alpha-neoendorphin, secretin, urokinase and plasminogen activating materials can be synthesized in microorganisms and animal cells.

In most cases, these materials are produced by using procaryotic E. coli as host cells. Recently, the use of eukaryotic yeast cells is drawing the attention of many researchers for such reasons as easy cultivation of yeast, rapid cell division, low hazard, and advanced analysis of the genetic and biochemical aspects of Saccharomyces yeast. However, no exogenous peptides have been produced in yeast except for alpha-interferon, hepatitis viral B surface antigen protein and alpha-neoendorphin.

The expressing of exogenous genes such as chemically synthesized genes, complementary DNA (cDNA) genes prepared from mRNA by reverse transcriptases, and genes prepared from chromosomes by a suitable treatment requires the presence of a promoter region adapted to the specific host cells, and the efficiency of the expressing of peptide genes greatly depends on the promoter used. Therefore, intensive efforts have been made to develop expressing plasmid vectors using various promoters.

However, the vectors so far reported for expressing exogenous genes using yeast cells as the host are not highly efficient in expressing the desired exogenous gene as compared with conventional vectors using E. coli cells as the host. Noting that glyceraldehyde-3-phosphate dehydrogenase (GAP-DH) which is an enzyme controlling the glycolysis of Saccharomyces is produced in yeast cells in a large amount, the present inventors thought that good results would be obtained by using a promoter (gene) for the operon coding for this enzyme. As a result of our studies made on the basis of this observation, we found that this promoter was very useful in the production of exogenous or endogenous peptides in the yeast. We then prepared a yeast vector having an exogenous or endogenous peptide gene inserted at a site downstream of the promoter, and used the so prepared vector in transforming the yeast. By analyzing the separated yeast cells, we confirmed that they had been transformed. This has lead to the accomplishment of the present invention.

Figs. 1 to 3 illustrate the sequence of constructing a plasmid bearing both GAP-DH gene and αNE gene.

Fig. 1 specifically shows the cloning of GAP-DH gene.

Fig. 2 shows the method of constructing a plasmid (pYαNE53) bearing both GAP-DH gene and αNE gene.

Fig. 3 shows the method of constructing a plasmid having the 3' terminal non-translational region of GAP-DH gene inserted into pYαNE53 at a point downstream of the αNE gene.

The yeast vector according to the present invention is prepared by the following procedure. Nucleic DNA in Saccharomyces XS 16-5C (cir°) cells is cleaved by a restriction enzyme HindIII to produce a DNA fragment having a length of 1.9 to 2.2 kb. This fragment is cloned to a Hind III site of a suitable vector plasmid, say, plasmid pBR322, to obtain a yeast gene library. From this library, a clone is obtained which has a yeast DNA fragment having a length of 2.1 kb, one SalI cleavage site and one HpaI cleavage site. The DNA fragment in this plasmid is determined for its base sequence by the Maxam-Gilbert method [PNAS 74, 560-564 (1977)]. By comparing the results with the data reported in Holland et al., "J. Biol. Chem.", 254, 9839 (1979), the insertion of the GAP-DH gene can be confirmed.

The plasmid having the desired GAP-DH gene is cut with a specific restriction enzyme, and a DNA fragment harboring the GAP-DH gene is inserted into a yeast vector carrying a selection marker, preferably E. coli-yeast shuttle vector which is used only for the purpose of facilitating the subsequent manipulation.

Then, a fragment bearing the target peptide gene is inserted into the vector in such a manner that the reading frame fits the SalI cleavage site in the vicinity of C terminal of the GAP-DH gene. The resulting plasmid carrying both the GAP-DH gene and the target peptide gene is used to transform yeast cells.

For the purposes of the present invention, the target peptide gene includes both an exogeous-peptide gene and an endogenous peptide gene in the yeast other than the GAP-DH gene.

We confirmed that the GAP-DH gene promoter in the transformed yeast is capable of expressing the target peptide gene in a very efficient manner.

It is known that the production of the target peptide in yeast can be significantly increased by incorporating 3'-terminal non-translational region of the GAP-DH gene in the plasmid at a point downstream of the target peptide gene. The effect of adding the 3'-terminal non-translational region is specifically described in Japanese Patent Application No. 167615/81. The method of using this 3'-terminal non-translational region, a plasmid having this region inserted downstream the target peptide gene, and a method of using such a plasmid are also included in the scope of the present invention.

The present invention is described in greater detail by reference to the following non-limiting example. In the example, alpha-neoendorphin (αNE) which is the target peptide was produced as GAP-αNE chimeric protein from sequences such as GAP-DH promoter-GAP-DH structual gene-αNE gene (abbreviated as pGAP-GAP-αNE) and pGAP-GAP-αNE-3'GAP

(with the 3'-terminal non-translational region of the GAP-DH gene attached), wherein αNE gene was the target peptide gene. Another target peptide, for example, interferon can be directly produced by attaching a gene coding for the target peptide to the GAP-DH promoter with a suitable base sequence being interposed. Therefore, the GAP-DH promoter can be extensively used not only to express various exogenous genes but also to produce exogenous or endogenous peptides other than alpha-neoendorphin.

Enxample

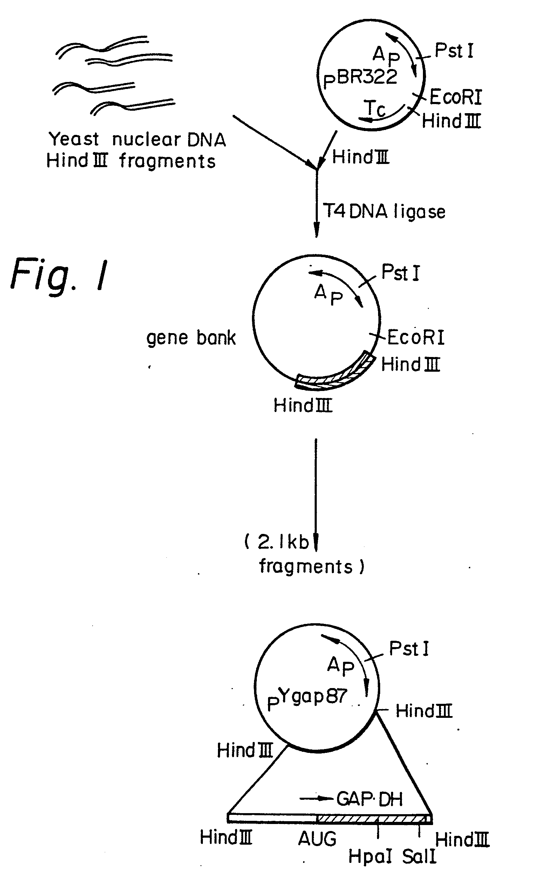

1. Cloning of GAP-DH gene (see Fig. 1)

Saccharomyces XS 16-5C (cir°) cells [Saccharomyces cerevisiae XS 16-5C MATa leu2 his3 trpl (sir°)] were cultured in 1,000 ml of YPD medium (1% yeast extract, 2% polypeptone and 2% glucose) at 30°C for 24 hours, and all DNA were separated from the cultured cells by the method of Cryer et al. described in "Isolation of Yeast DNA", Methods in Cell Biology, vol. 12, Academic Press, New York, 1975, pp. 39-44.

The yeast DNA (50 μg) was cleaved by HindIII (100 units) by heating at 37°C for 2 hours. The reaction solution was subjected to 0.8% agarose gel electrophoresis, and the obtained DNA HindIII fragments having a length of 1.9-2.2 kb were purified. Plasmid pBR322 (0.5 μg) was cleaved with HindIII (1 unit) by reaction in TA buffer (33 mM tris-$CH_3COOH$ buffered at pH 7.9, 66 mM $CH_3COOK$, 10 mM$(CH_3COO)_2 \cdot Mg$, 0.5 mM DTT) at 37°C for 1 hour. The two kinds of DNA were dissolved in T4 DNA ligase buffer, (20 mM tris-HCl buffered at pH 7.5, 10 mM $MgCl_2$, 10 mM DDT and 0.5 mM ATP) and after adding 2 units of T4 DNA ligase, and subjected to ligation at 15°C for 18 hours. The reaction solution (10 μl) was added to E. coli JA221 that had been treated with $CaCl_2$ (0.3 ml). From the transformed ampicillin-resistant clones, 400 clones which were sensitive to tetracycline were selected and analyzed for the plasmid DNA by the alkali denaturation method.

According to the analytical data reported by Holland et al., a 2.1 kb fragment bearing the GAP-DH gene has two restriction sites, one by HpaI and the other by SalI.

A 2.1 kb HindIII fragment is cleaved into two sub-fragments (0.14 kb and 1.96 kb) by SalI, and is also cut into two sub-fragments (1.39 kb and 0.71 kb) by HpaI.

Our analysis of the 400 clones which were resistant to ampicillin and sensitive to tetracycline showed that one of them contained a plasmid having the same restriction sites as in the case of the above reported fragment containing GAP-DH gene. We named this plasmid pYgap87.

The plasmid pYgap87 was cut by HindIII at a point closer to Sal I restriction site, and the resulting cohesive end was labelled with gamma-$[^{32}P]$-ATP and polynucleotide kinase, and the DNA base sequence was determined by the Maxam-Gilbert method. By comparing the results with the data of Holland et al., we confirmed that pYgap87 had the GAP-DH gene. E. coli K-12 cells that were transformed by pYgap87 were named E. coli SBM 151 and have been deposited with the Fermentation Research Institute, Japan (deposite No. FERM P-6762; International Deposition Acceptance No. FERM BP-382 under the Budapest Treaty).

2. Preparation of pYE237 plasmid vector

Plasmid vector pYE237 is the same as pYE227 vector described in Japanese Patent Application No. 167615/81 except that the former does not contain SalI cleavage site. This plasmid was prepared by the following method.

Plasmid pYE227 (5 µg) was cleaved by SalI (10 units) by heating in TA buffer at 37°C for 1.5 hours. By subsequent heating at 65°C, SalI was inactivated, and thereafter, 0.3 mM of four kinds of dNTP and 8 mM of 2-mercaptoethanol were added. The solution was heated at 37°C for 30 minutes in the presence of T4 DNA polymerase (1 unit) so as to fill in the cohesive ends produced by cleavage with SalI. After one extraction with phenol, DNA was precipitated by ethanol twice the volume of the reaction solution. The DNA precipitate was dissolved in T4 DNA ligase buffer (20 µl), and subjected to ligation at 15°C for 18 hours in the presence of T4 DNA ligase (5 units).

The reaction solution was introduced into E. coli JA 221 as donor DNA according to the conventional method.

From the transformed ampicillin-resistant clones, DNA was separated and analyzed for confirming the production of pYE 237 having no SalI restriction site.

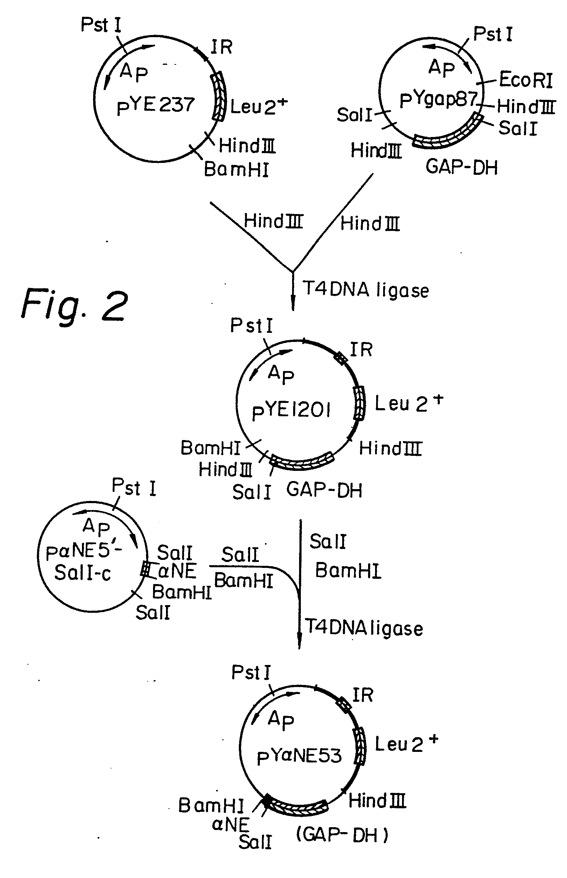

3.  Preparation of pYE1201 yeast-E. coli shuttle vector (see Fig. 2)

Plasmid pYE237 (5 μg) was cleaved with HindIII by heating in TA buffer at 37°C for 1 hour. Throughout the example, all reactions with restriction enzymes were conducted in TA buffer at 37°C for 1 hour. The already prepared plasmid pYgap87 (5 μg) was cleaved with HindIII (20 units) and subjected to agar electrophoresis to separate 2.1 kb DNA fragments (GAP-DH gene), followed by elution and purification of the DNA. The purified 2.1 kb DNA fragments and DNA fragments obtained by cutting pYE237 with HindIII were dissolved in DNA 20 μl of DNA ligase buffer and subjected to ligation at 15°C for 16 hours in the presence of T4 DNA ligase (1 unit). The reaction solution (10 μl) was introduced into $CaCl_2$ (0.3 ml)-treated E. coli JA221 so as to transform the latter.

Plasmid DNA was isolated from the ampicillin-resistant transformants by the conventional method and analyzed for confirming the production of pYE1201.

Yeast (XS 16-5C) cells transformed by this plasmid were named Saccharomyces cerevisiae SBM 321 and have been deposited with the Fermentation Research Institute (deposit No. FERM P-6766; International Deposition Acceptance No. FERM BP-381 under the Budapest Treaty).

4.  Preparation of plasmid DNA bearing αNE gene (pαNE5'-SalI-c)

According to the method described in Nucleic Acid Research, 10, 1741-1754 (1982), an αNE gene (EcoRI-BamHI DNA fragment) made of 46 base pairs was prepared. The αNE gene was inserted into the larger fragment (4 kb) of the two fragments obtained by cutting pBR322 with EcoRI and Bam HI. By the procedure described below, SalI linker composed of 5'-GGGTCGACCC-3' was inserted to the resulting pYαNE5' to form plasmid pYαNE5'-(SalI)-c. This was for the purpose of inserting αNE so that the reading frame would fit Sal I site in the GAP-DH gene. Plasmid pYαNE5' (5 μg) was cleaved with EcoRI (10 units) by heating in TA buffer at 37°C for 1 hour. After additional heating at 65°C, 0.3 mM of two kinds of dNTP (dATP and dTTP) and 8 mM of 2-mercaptoethanol were added, and the solution was heated at 37°C for 30 munites to fill in the cohesive ends formed by cleavage with EcoRI. After one extraction with phenol, DNA was recovered by precipitation with ethanol. SalI linker (1 μg) had its 5'-terminal phosphorylated by heating in a buffer (50 mM tris-HCl buffered at pH 7.5 and 10 mM $MgCl_2$) at 37°C for 45 minutes in the presence of ATP (25 nmol) and polynucleotide kinase (10 units). The two DNA fractions were mixed and reacted in T4 DNA ligase buffer at 15°C for 18 hours in the presence of T4 DNA ligase (5 units). The reaction solution (10 μl) was introduced into $CaCl_2$ (0.3 ml)-treated E. coli JA221. Plasmid DNA was separated from the ampicillin-resistant transformants by the conventional manner and analyzed to confirm the construction of pYαNE-5'-SalI-c.

5. <u>Insertion of alpha-neoendorphin (αNE) gene (see Fig. 2)</u>

Plasmid pYE1201 (5 μg) was cut into two fragments by BamHI (20 units) and SalI (20 units) and the two fragments were separated by agar electrophoresis. The larger fragment was eluted from the agar and purified. Plasmid pαNE5'-SalI-c (50 μg) was also cut into two fragments by BamHI (100 units) and SalI (100 units) and the two fragments were separated by 5% polyacrylamide gel electrophoresis. A DNA fragment made of 50 base pairs and containing the αNE gene was eluted from the gel and purified. The two DNA fragments were dissolved in DNA ligase buffer (20 μl) and heated at 15°C for 16 hours in the presence of T4 DNA ligase (2 units). The reaction solution (10 μl) was introduced into $CaCl_2$ (0.3 ml)-treated E. coli JA221. Plasmid DNA was separated from the ampicillin-resistant transformants by the conventional manner and analyzed to confirm the production of pYαNE53.

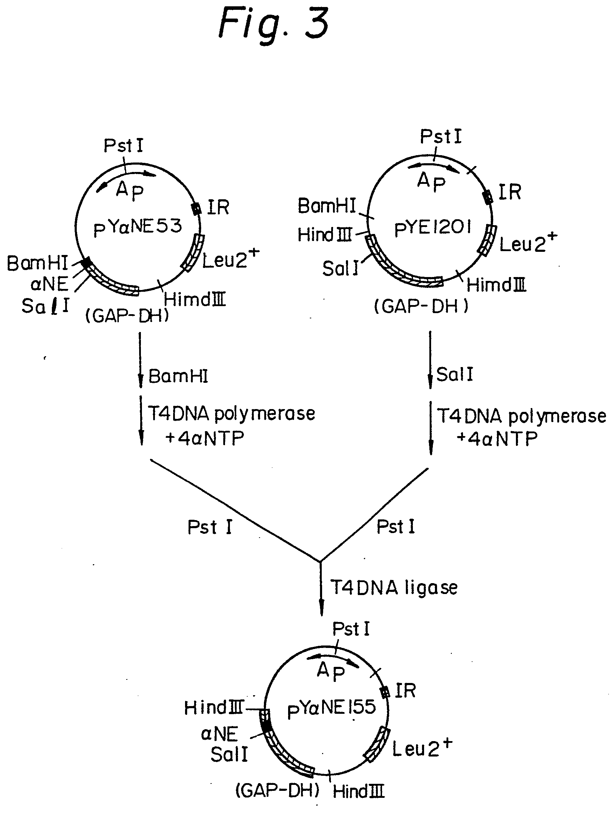

6. <u>Addition of 3'-terminal region of GAP-DH gene to pYαNE53 (see Fig. 3)</u>

The 3'-terminal region of GAP-DH gene was introduced

into pYαNE53 at a point downstream of the αNE gene in the following manner.

Plasmid pYαNE53 (10 µg) was cut with BamHI (30 units) and heated in a T4 DNA polymerase buffer (67 mM tris-HCl buffered at pH 8.8, 6.7 mM $MgCl_2$, 10 mM 2-mercaptoethanol, 16.6 mM ammonium sulfate, 6.7 µM EDTA, 0.3 mM of four kinds of dNTP) at 37°C for 30 minutes in the presence of T4 DNA polymerase (1 unit) so as to fill in the cohesive ends formed by cutting with BamHI.

The 3'-terminal region of GAP-DH gene was obtained from pYE1201 by the following procedure. Plasmid pYE1201 (10 µg) was cut with SalI (30 units) and heated in a T4 DNA polymerase buffer at 37°C for 30 minutes in the presence of T4 DNA polymerase (1 unit) so as to fill in the cohesive ends formed by cutting with SalI. The two plasmids were cleaved with PstI (30 units), and by agarose electrophoresis, a fraction containing αNE gene was separated from pY NE53, and a fraction containing the 3'-terminal region of GAP-DH gene was separated from pYE1201, and the two fractions were purified. They were ligated to each other by reacting in T4 DNA ligase buffer (20 µl) at 15°C for 17 hours in the presence of T4 DNA ligase (5 units). The reaction solution (10 µl) was introduced into $CaCl_2$ (0.3 ml)-treated E. coli JA221. Plasmid DNA was separated from the ampicillin-resistant transformants by the conventional manner and analyzed to confirm the construction of pYαNE155.

7.  Transformation and culture of yeast

Plasmids pYE1201, pYαNE53 and pYαNE155 were introduced into Saccharomyces cerevisiae XS 16-5C according to the method described in Beggs J.D., Nature, 27, 5104 (1978). The resulting transformants were shake-cultured in a YPD medium (1% yeast extract, 2% polypeptone and 2% glucose) at 30°C for 24 hours and the cells were recovered by centrifugation. Cells recovered from 1 ml of the culture were mixed with 0.5 ml of cold acetone and the mixture was left to stand at -20°C for 1 - 24 hours, and freeze-dried. The dried cells were suspended in 70% formic acid containing cyanogen bromide (5 mg/ml) and the suspension was left to stand in a dark place for 24 hours. The suspension was freeze-dried and αNE was eluted with 0.1N acetic acid (0.1 ml). The eluted αNE was neutralized with 1.3M tris-HCl (pH 8.0) and subjected to radioimmunoassay (RIA) according to the method described in N. Minamino et al., BBRC, vol. 102, 226 (1981). The results are shown in Table 1. Plasmid pYαNE53 (having no 3'-terminal region of GAP-DH gene) expressed two million molecules per cell of chimeric protein having αNE attached right after the N-terminal (323 rd amino acid residue) of GAP-DH Plasmid pYαNE155 (having 3'-terminal region of GAP-DH gene attached to pYαNE53) expressed 20 million molecules of the same chimeric protein per cell. This was equivalent to the productivity of about 2 μg of the chimeric protein per ml of the culture. To determine the stability of the three plasmids, they were cultured through 10 generations under non-selective conditions in YPD medium. The stability factor of pYE1201 having a complete GAP-DH was relatively low (10-20%). On the other hand, pYαNE53 and pYαNE155 having the αNE gene attached in the vicinity of C-terminal of GAP-DH has somewhat imporved stability.

## Table 1

Productivity of αNE in the presence of GAP-DH promoter

| Plasmid | Cell count per 1 ml | αNE productivity | | Plasmid stability |
| --- | --- | --- | --- | --- |
| | | ng/ml | no. of mole-culesper cel | |
| pYE1201 | $1.83 \times 10^8$ | <0.01 | – | 15.1% |
| pYαNE53 | $1.28 \times 10^8$ | 445 | $1.73 \times 10^6$ | 42.0% |
| | $0.6 \times 10^8$ | 337 | $2.8 \times 10^6$ | 78.9% |
| | $1.12 \times 10^8$ | 368 | $1.6 \times 10^6$ | N.D.* |
| pYαNE155 | $0.99 \times 10^8$ | 2049 | $1.0 \times 10^7$ | 43.2% |
| | $0.20 \times 10^8$ | 702 | $1.7 \times 10^7$ | N.D.* |
| | $0.25 \times 10^8$ | 1260 | $2.5 \times 10^7$ | 25.5% |
| | $0.42 \times 10^8$ | 1706 | $2.0 \times 10^7$ | N.D.* |

* Not measured.

Claims:

1.      A plasmid containing a promoter for the gene controlling glyceraldehyde-3-phosphate dehydrogenase and which
expresses a gene for the target peptide in yeast.

2.      A process for producing the target peptide by preparing
a plasmid containing a promoter for the gene controlling
glyceraldehyde-3-phosphate dehydrogenase and a gene for the
target peptide, and culturing a yeast that has been trans-
formed by said plasmid.

*Fig. 1*

*Fig. 2*

## Fig. 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | EP-A-0 060 057 (GENENTECH) <br> * Pages 15-22; page 25, line 19; claims 1-14 * <br> --- | 1,2 | C 12 N 15/00 |
| P,X | EP-A-0 077 689 (SUNTORY) <br> & JP - A - 16761581(Cat. D) * Page 3; page 25; pages 28-36; claims 1-8 * <br> --- | 1,2 | |
| P,X | EP-A-0 089 666 (GENENTECH) <br> * Claims 1-12; pages 7,8 * <br> --- | 1,2 | |
| E | EP-A-0 096 910 (UNILEVER) <br> * Claims 1-8; figures 1-4 * <br> --- | 1,2 | |
| E | EP-A-0 096 430 (GIST-BROCADES) <br> * Claims 1-38; figures 5,6; pages 30-34 * | 1,2 | |

TECHNICAL FIELDS SEARCHED (Int. Cl. ³)

C 12 N

-----

The present search report has been drawn up for all claims

| Place of search | Date of completion of the search | Examiner |
|---|---|---|
| THE HAGUE | 31-01-1984 | DELANGHE L.L.M. |